# EUROPEAN PATENT SPECIFICATION

(11) **EP 1 547 678 B1**
(45) Date of publication and mention of the grant of the patent: **13.07.2016**
(21) Application number: 04258031.6
(22) Date of filing: 22.12.2004
(51) Int. Cl.: B01J 19/00, B82Y 30/00, C07H 21/00, C07H 21/04, C40B 40/06, C40B 40/10, C40B 40/12, C40B 50/14, C40B 60/14

(54) **Process for high-yield synthesis of nucleic acid arrays**
Verfahren mit hoher Ausbeute zur Herstellung von Nukleinsäureanordnungen
Procédé à haut rendement pour la synthèse de matrices d'acides nucléiques

(30) Priority: 22.12.2003 US 532220 P; 03.06.2004 US 577050 P
(43) Date of publication of application: 29.06.2005
(73) Proprietor: Affymetrix, Inc., Santa Clara, CA 95051 (US)
(72) Inventor: McGall, Glenn H., California 94303 (US); Kuimelis, Robert G., California 94303 (US); Goldberg, Martin J., California 95070 (US); Xu, Guangyu, California 95035 (US); Parker, Nineveh, California 95037 (US)
(74) Representative: Boult Wade Tennant

(56) References cited:
- WO-A-00/75372
- WO-A-97/39151
- WO-A-98/20967
- WO-A-99/41007

## Description

### BACKGROUND OF THE INVENTION

Methods of synthesizing polymer sequences such as nucleotide and peptide sequences are known. Methods of synthesizing oligonucleotides are found in, for example, Oligonucleotide Synthesis: A Practical Approach, Gait, ed., IRL Press, Oxford (1984). The so-called "Merrifield" solid phase peptide synthesis has been in common use for many years and is discussed in Merrifield, J. Am. Chem. Soc. (1963) 85:2149-2154. Solid-phase synthesis techniques have been provided for the synthesis of polymers, including peptides and nucleic acids on, for example, a number of "pins." See e.g., Geysen et al., J. Immun. Meth. (1987) 102:259-274. Other solid-phase techniques involve, for example, synthesis of various peptide sequences on different cellulose disks supported in a column. See Frank and Doring, Tetrahedron (1988) 44:6031-6040. Still other solid-phase techniques are discussed in U.S. Patent No. 4,728,502 (issued to Hamill) and PCT Publication No. WO 90/00626 (Beattie, inventor). Techniques have also been developed for the photolithographic synthesis of high density polymer arrays, including high density nucleic acid arrays. One technique that has been commercially used to produce high density oligonucleotide arrays is the use of photoprotective groups to build up nucleic acids *in situ.* See WO 98/20967, WO 99/41007 and WO 00/75372.

### SUMMARY OF THE INVENTION

The invention provides a process for fabricating an array of polymers comprising: providing a solid substrate comprising a reactive group protected by a protective group; coating said solid substrate with a film, said film comprising a photoacid generator, a sensitizer and a base, wherein the film is spun coated onto the substrate; activating said photoacid generator in selected areas by selective application of light to produce an acid wherein a step of baking is not performed following activation of the photoacid generator; and exposing said reactive group having said protective group to said acid under appropriate conditions such that said protecting group is removed to provide a monomer with an exposed reactive group wherein said step of exposure does not result in substantial damage to said polymers of the array. In preferred embodiments of the disclosed invention, the array of polymers is an array of nucleic acids or an array of oligonucleotides.

The present invention discloses the monomer in the process is preferably a nucleotide or amino acid. It is also disclosed that a nucleotide is preferably protected with a DMT protecting group at its 5' or 3' hydroxyl moiety. In accordance with the present invention, it is also disclosed that the monomer is preferably an amino acid which is preferably protected by a tBOC protective group at its amino terminal end.

In accordance with the present invention the activatable deprotecting agent is a photoacid generator. In preferred embodiments of the present invention, the photoacid generator is 2,6-dinitrobenzyl tosylate. In another preferred embodiment of the present invention, the photoacid generator is an onium salt. In the case of photoacid generators, the activator is light. The light preferably has a wavelength of about 330 to 365 nm. In accordance with the present invention, no post-photo exposure baking step is performed. In accordance with the present invention, it has been discovered that such baking or heating is destructive of nucleic acid polymers, e.g., causes depurination.

In still other embodiments of the present invention, the film contains the polymer poly(methyl methacrylate).

In still other embodiments of the present invention, the method employs additional steps of reacting the exposed reactive group with a protected monomer. The present invention discloses that these steps may be further repeated until the desired polymer array is fabricated. The present invention discloses that the array is preferably comprised of a polymer of between 20 to 75 monomers in length.

### DETAILED DESCRIPTION OF THE INVENTION

### DEFINITIONS

As used herein, the following terms are intended to have the following general meanings:
Base: A base is an alkaline compound which may be used in conjunction with certain photoacid generators in accordance with the present invention. Examples of bases in accordance with an aspect of the present invention include N-octylamine and di-t-butyl aniline. While applicants disclaim being held to any particular mechanistic theory, in accordance with an aspect of the present invention, the base is used a contrast enhancer. The base may act as a buffer, neutralizing, for example, the first mole equivalent of acid that's generated by the PAG. By doing this, small amounts of acid that may be generated due to stray light from the imaging system will not cause any detritylation response on the substrate where the monomer is a DMT protected monomer. In effect a "threshold" level of acid must be generated before free acid can build up in the film and detritylation can occur. High-resolution imaging systems tend to have lower contrast (i.e., edge resolution profiles are sharp, but dark areas are not totally dark). Bases also serve to protect against small "background" amounts of acid that may occur from impurities in the PAG reagent or from its thermal decomposition on storage or during processing, etc.

Film: A film as used herein refers to a layer or coating having one or more constituents, applied in a generally uniform manner over the entire surface of the substrate for example by spin coating. For example, in accordance with an aspect of the present invention, a film is, for example, a solution, suspension, dispersion, emulsion, etc., of a chosen polymer, including a photoacid generator, a base and a sensitizer.

Ligand: A ligand is a molecule that is recognized by a receptor. Examples of ligands that can be investigated by at least one aspect of the present invention include, but are not restricted to, agonists, antagonists, toxins, receptors, venoms, viral epitopes, hormones, opiates, steroids, peptides, enzyme substrates, cofactors, drugs, lectins, sugars, oligonucleotides, nucleic acids, oligosaccharides, and proteins.

Monomer: A monomer is a member of the set of small molecules which are or can be joined together to form a polymer or a compound composed of two or more members. The set of monomers includes but is not restricted to, for example, the set of common L-amino acids, the set of D-amino acids, the set of synthetic and/or natural amino acids, the set of nucleotides, and the set of pentoses and hexoses, each set of which is readily known to those of skill in the art. The particular ordering of monomers within a polymer is referred to herein as the "sequence" of the polymer. As used herein, "monomers" refers to any member of a basis set for synthesis of a polymer, and is not limited to a single "mer". For example, dimers of the 20 naturally occurring L-amino acids form a basis set of 400 monomers for synthesis of polypeptides. Monomers can also include trimers, oligomers, polymers and so forth. Different basis sets of monomers may be used at successive steps in the synthesis of a polymer. Furthermore, each of the sets may include protected members composed of protected amino acids, as described above. Other examples of polymers within the scope of the present invention include without limitation linear and cyclic polymers of nucleic acids, polysaccharides, phospholipids, and peptides having either α-, β-, or ω-amino acids, heteropolymers in which a known drug is covalently bound to any of the above, polynucleotides, polyurethanes, polyesters, polycarbonates, polyureas, polyamides, polyethyleneimines, polyarylene sulfides, polysiloxanes, polyimides, polyacetates, or other polymers within the scope of the present invention as would be understood by a person of kill in the art of this disclosure. Such polymers are "diverse" when polymers having different monomer sequences are formed at different predefined regions of a substrate. Methods of cyclization and polymer reversal are disclosed in U.S. Patent No. 5,242,974.

Peptide: A peptide is a polymer in which the monomers are α-amino acids and are joined together through amide bonds, alternatively referred to as a polypeptide. Amino acids may be in the L-optical isomer form or the D-optical isomer form. The term "polypeptide" as used herein refers to two or more amino acid monomers in length or greater and often includes more than 20 amino acid monomers or monomers on the order of hundreds. Standard abbreviations for amino acids are used (e.g., P for proline). Identification of amino acids and their abbreviations are well-known and are included in Stryer, Biochemistry, Third Ed., 1988.

Receptor: A receptor is a molecule that has a specific affinity for a ligand and usually binds tightly to the ligand. Receptors may be naturally occurring or synthetic molecules. Ligands can be employed in their unaltered state or as aggregates with other molecules. Receptors may be attached, covalently or noncovalently, to a binding member or ligand, either directly or via a specific binding substance. Examples of receptors which can be employed by this invention include, but are not restricted to, antibodies, cell membrane receptors, monoclonal antibodies and antisera reactive with specific antigenic determinants, viruses, cells, drugs, polynucleotides, nucleic acids, peptides, cofactors, lectins, sugars, polysaccharides, cellular membranes, and organelles. Receptors are sometimes referred to in the art as antiligands. A "Ligand Receptor Pair" is formed when two molecules (e.g. a ligand and a receptor) have combined through molecular recognition to form a complex. Specific examples of receptors which can be investigated by this invention include but are not restricted to:
a.) Microorganism receptors: The determination of ligands that bind to microorganism receptors such as specific transport proteins or enzymes essential to survival of microorganisms would be a useful tool for discovering new classes of antibiotics. Of particular value would be antibiotics against opportunistic fungi, protozoa, and bacteria resistant to antibiotics in current use.
b.) Enzymes: A receptor can comprise a binding site of an enzyme such as an enzyme responsible for cleaving a neurotransmitter; determination of ligands for this type of receptor to modulate the action of an enzyme that cleaves a neurotransmitter is useful in developing drugs that can be used in the treatment of disorders of neurotransmission.
c.) Antibodies: For instance, one aspect of the instant invention may be useful in investigating a receptor that comprises a ligand-binding site on an antibody molecule which combines with an epitope of an antigen of interest; analyzing a sequence that mimics an antigenic epitope may lead to the development of vaccines in which the immunogen is based on one or more of such sequences or lead to the development of related diagnostic agents or compounds useful in therapeutic treatments such as for autoimmune diseases (e.g., by blocking the binding of the "self" antibodies).
d.) Nucleic Acids: Nucleic acids may be synthesized to establish sequences recognized by various receptor molecules, such as protein or other DNA or RNA molecules. Nucleic acids within the scope of the present invention include naturally occurring or synthetic bases or monomers, nucleic acid analogs, modified nucleic acids, nucleic acids containing modified nucleotides, modified nucleic acid analogs, oligonucleotides of whatever length, peptide nucleic acids and the like or mixtures thereof.
e.) Catalytic Polypeptides: Typically referred to as enzymes act to catalyse particular chemical reactions involving the conversion of one or more reactants to one or more products. Enzymes generally include a binding site specific for at least one reactant or reaction intermediate and an active functionality proximate to the binding site, which functionality is capable of chemically modifying the bound reactant. Catalytic polypeptides and others are discussed in, for example, PCT Publication No. WO 90/05746, WO 90/05749, and WO 90/05785.
f.) Hormone receptors: Determination of a ligand which binds with high affinity to a receptor such as the receptors for insulin and growth hormone is useful in the development of, for example, an oral replacement of the daily injections which diabetics must take to relieve the symptoms of diabetes or a replacement for growth hormone. Other examples of hormone receptors include the vaso-constrictive hormone receptors; determination of ligands for these receptors may lead to the development of drugs to control blood pressure.

Sensitizer: A sensitizer is a compound which aids in the use of certain photoacidgenerators ("PAGs"). The sensitizer aids in this process by reacting with the energy source to initiate the photo-reaction of the PAG. For certain applications of the present invention, it is desirable to extend the photosensitivity of the PAG. One approach to this would be to add an appropriate chromophore into the structure of the PAG. Yet another approach to this issues in accordance with the present invention, is to add a sensitizer to the photoresist, also called a photosensitizer, which is capable of activating the PAG at, for example, a longer wavelength of light.

Substrate: A material having a rigid, semi-rigid or gelatinous surface. Typical examples include glass or suitable polymer materials. In some embodiments of the present invention, at least one surface of the substrate will be substantially flat, although in some embodiments it may be desirable to physically separate synthesis regions for different polymers with, for example, wells, raised regions, etched trenches, or the like. In some embodiments, the substrate itself contains wells, trenches, flow through regions, etc. which form all or part of the synthesis regions. According to other-embodiments, small beads may be provided on the surface, and compounds synthesized thereon optionally may be released upon completion of the synthesis. Substrates are well known in the art and are readily commercially available through vendors such as USPG, PPG Industries, AFG Industries and others.

Protective Group: A group or moiety which may be selectively removed to expose an active site such as an amino functionality in peptide or amino acid or a hydroxyl group in a nucleic acid or nucleotide. In accordance with one aspect of the present invention, protective groups may be removed under a variety of condition, for example, depending on the nature of the protective group and the mode of its connection to the active sites basic or acidic conditions may be employed as appropriate. For an extensive listing of protective groups useful in the practice of the present invention, see also Greene, T.W. and Wuts, P.G.M., Protective Groups in Organic Synthesis, (1991). Useful representative acid sensitive protective groups include dimethoxytrityl (DMT), tert-butylcarbamate (tBoc) and trifluoroacetyl (tFA). Useful representative base sensitive protective groups include 9-fluorenylmethoxycarbonyl (Fmoc), isobutyrl (iBu), benzoyl (Bz) and phenoxyacetyl (pac). Other protective groups include acetamidomethyl, acetyl, tert-amyloxycarbonyl, benzyl, benzyloxycarbonyl, 2-(4-biphenylyl)-2-propyloxycarbonyl, 2-bromobenzyloxycarbonyl, tert-butyl, tert-butyloxycarbonyl, 1-carbobenzoxamido-2,2,2-trifluoroethyl, 2,6-dichlorobenzyl, 2-(3,5-dimethoxyphenyl)-2-propyloxycarbonyl, 2,4-dinitrophenyl, dithiasuccinyl, formyl, 4-methoxybenzenesulfonyl, 4-methoxybenzyl, 4-methylbenzyl, o-nitrophenylsulfenyl, 2-phenyl-2-propyloxycarbonyl, α-2,4,5-tetramethylbenzyloxycarbonyl, p-toluenesulfonyl, xanthenyl, benzyl ester, N-hydroxysuccinimide ester, p-nitrobenzyl ester, p-nitrophenyl ester, phenyl ester, p-nitrocarbonate, p-nitrobenzylcarbonate, trimethylsilyl and pentachlorophenyl ester and the like.

Predefined Region: A predefined region is a localized area on a substrate which is, was, or is intended to be used for formation of a selected polymer and is otherwise referred to herein in the alternative as "reaction" region, a "selected" region, simply a "region" or a feature. The predefined region may have any convenient shape, e.g., circular, rectangular, elliptical, wedge-shaped, etc. In accordance with the present invention, the arrays of the present invention have features on the order of 10-100 µm, i.e. 10 x 10 µm² to 100 x 100 µm² for approximately square features. More preferably the features will be on the order of 1-10 µm. It is also an object of the present invention to provide features having submicron dimensions. Such features are preferably on the order of 100-1000 nm. Within these regions, the polymer synthesized therein is preferably synthesized in a substantially pure form. However, in other embodiments of the invention, predefined regions may substantially overlap. In such embodiments, hybridization results may be resolved by software for example.

A Deprotecting Agent is a chemical or agent which causes a Protective Group to be cleaved from, for example, a protected monomer. Such cleavage, in accordance with the present invention, preferably exposes a reactive group on the monomer. The reactive group may then, in accordance with the present invention, be used to couple the deprotected monomer to the next monomer creating the polymer step by step using the appropriate chemistry. This next monomer coupled would, in accordance with one aspect of the present invention, bear a protective group which could in turn be cleaved under appropriate conditions.

An Activatable Deprotecting Agent is a chemical or agent which is relatively inert with respect to a Protective Group bound to a monomer, i.e., the activatable deprotecting agent will not cause cleavage of the protective group in any significant amount absent activation. An activatable deprotecting agent may be activated in a variety of ways depending on it's chemical and physical properties. Certain activatable deprotecting agents may be activated by exposure to some form of activator, e.g. electromagnetic radiation. An activatable deprotecting agent may be activatable at only certain wave lengths of electromagnetic radiation and not at others. For example, certain activatable deprotecting reagents will be activated with visible or UV light.

Damage to the polymer: it is an object of the present invention that the reagents and conditions used to deprotect the monomer, whether attached to a linker or growing polymer chain, do not substantially degrade or harm the polymer, monomer, linker or substrate. Preferably, the reagents and conditions used to deprotect will not damage the polymer at all or will do so only minimally such that the polymer can still be specifically recognized by its counterpart (e.g. ligand-receptor). For example, if the polymer is a nucleic acid, it can only sustain damage, e.g., depurination, to the extent that it can still undergo specific Watson-Crick base pairing with a complementary nucleic acid such that specific hybridization is detectable over non-specific hybridizations. Acceptable levels of damage will be readily appreciated by those of skill in the art. In constructing an array of polymers in accordance with the present invention, it is acceptable that some polymers of a group are extensively damaged as long as there are sufficient other members of the group that are either undamaged or minimally damaged to allow specific recognition of the polymer.

A Photoacid Generator is a compound or reagent which produces an acid upon treatment with electro magnetic radiation (e.g. light) of a selected wave length.

As used in this application, the singular form "a," "an," and "the" include the corresponding plural references unless the context dictates otherwise. Likewise, plural references include the singular unless the context indicates otherwise.

Throughout this disclosure, various aspects of this invention can be presented in a range format. It should be understood that such description is merely for convenience and brevity and should not be construed as an unwarranted limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques of organic chemistry, polymer technology, molecular biology (including recombinant nucleic acid techniques), cell biology, biochemistry, and immunology as would be understood by one of the ordinary skill. Such conventional techniques include polymer array synthesis, hybridization, ligation, and detection of a hybridization using a label. Specific illustrations of suitable techniques can be had by reference to the examples herein below. However, other equivalent conventional procedures can, of course, also be used. Such conventional techniques and descriptions can be found in standard laboratory manuals such as Genome Analysis: A Laboratory Manual Series (Vols. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press), Stryer, L. (1995) Biochemistry (4th Ed.) Freeman, New York, Gait, "Oligonucleotide Synthesis: A Practical Approach" 1984, IRL Press, Lond*on,* Nelson and Cox (2000), Lehninger, Principles of Biochemistry 3rd Ed., W.H. Freeman Pub., New York, NY and Berg et al. (2002) Biochemistry, 5th Ed., W.H. Freeman Pub., New York, NY.

Methods and techniques applicable to polymer (including protein) array synthesis have been described in U.S. Serial No. 09/536,841, WO 00/58516, U.S. Patent Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in International Publication Number WO 99/36760 and International Publication Number WO 01/58593.

Patents that describe synthesis techniques in specific embodiments include U.S. Patent Nos. 5,412,087, 6,147,205, 6,262,216, 6,310,189, 5,889,165, and 5,959,098. Nucleic acid arrays are described in many of the above patents, but the same general methodologies are applicable to polypeptide arrays.

There are many uses for polymers attached to solid substrates. These uses include gene expression monitoring, profiling, library screening, genotyping and diagnostics. Gene expression monitoring, and profiling methods can be shown in U.S. Patent Nos. 5,800,992, 6,013,449, 6,020,135, 6,033,860, 6,040,138, 6,177,248, and 6,309,822. Genotyping and uses therefore are shown in U.S. Patent Application Publication 20030036069, and U.S. Patent Nos. 5,856,092, 6,300,063, 5,858,659, 6,284,460, 6,361,947, 6,368,799 and 6,333,179.
Other uses are embodied in U.S. Patent Nos. 5,871,928, 5,902,723, 6,045,996, 5,541,061, and 6,197,506.

There are described herein sample preparation methods. Prior to or concurrent with genotyping, the genomic sample may be amplified by a variety of mechanisms, some of which may employ PCR. See, e.g, PCR Technology: Principles and Applications for DNA Amplification (Ed. H.A. Erlich, Freeman Press, NY, NY, 1992); PCR Protocols: A Guide to Methods and Applications (Eds. Innis, et al., Academic Press, San Diego, CA, 1990); Mattila et al., Nucleic Acids Res. 19, 4967 (1991); Eckert et al., PCR Methods and Applications 1,17 (1991); PCR (Eds. McPherson et al., IRL Press, Oxford); and U.S. Patent Nos. 4,683,202, 4,683,195, 4,800,159 4,965,188,and 5,333,675. The sample may be amplified on the array. See, for example, U.S. Patent No. 6,300,070.

Other suitable amplification methods include the ligase chain reaction (LCR) (*e.g.,* Wu and Wallace, Genomics 4,560 (1989), Landegren et al., Science 241, 1077 (1988) and Barringer et al. Gene 89:117 (1990)) transcription amplification (Kwoh et al., Proc. Natl. Acad. Sci. USA 86, 1173 (1989) and WO88/10315), self-sustained sequence replication (Guatelli et al., Proc. Nat. Acad. Sci. USA, 87, 1874 (1990) and WO90/06995), selective amplification of target polynucleotide sequences (U.S. Patent No 6,410,276), consensus sequence primed polymerase chain reaction (CP-PCR) (U.S. Patent No. 4,437,975), arbitrarily primed polymerase chain reaction (AP-PCR) (U.S. Patent No. 5,413,909, 5,861,245) and nucleic acid based sequence amplification (NABSA). (See, U.S. Patent Nos. 5,409,818, 5,554,517, and 6,063,603). Other amplification methods that may be used are described in, U.S. Patent Nos. 5,242,794, 5,494,810, 4,988,617 and in U.S. Serial No. 09/854,317.

Additional methods of sample preparation and techniques for reducing the complexity of a nucleic sample are described in Dong et al., Genome Research 11, 1418 (2001), in U.S. Patent No. 6,361,947, 6,391,592 and
U.S. Patent Application Publication 20030096235, U.S. Patent Application Publication 20030082543, and U.S. Patent Application Publication 20030036069.

Numerous methods for conducting polynucleotide hybridization assays have been well developed. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y, 1989); Berger and Kimmel Methods in Enzymology, Vol. 152, Guide to Molecular Cloning Techniques (Academic Press, Inc., San Diego, CA, 1987); Young and Davism, P.N.A.S, 80:1194 (1983). Methods and apparatus for carrying out repeated and controlled hybridization reactions have been described in U.S. Patent Nos. 5,871,928, 5,874,219, 6,045,996 and 6,386,749, 6,391,623.

Hybridization between a ligand and its corresponding receptor can be detected by generation of specific signals. See U.S. Patent Nos. 5,143,854, 5,578,832; 5,631,734; 5,834,758; 5,936,324; 5,981,956; 6,025,601; 6,141,096; 6,185,030; 6,201,639; 6,218,803; and 6,225,625, and in WO99/47964.

Methods and apparatus for signal detection and processing of intensity data are disclosed in, for example, U.S. Patents Nos. 5,143,854, 5,547,839, 5,578,832, 5,631,734, 5,800,992, 5,834,758, 5,856,092, 5,902,723, 5,936,324, 5,981,956, 6,025,601, 6,090,555, 6,141,096, 6,185,030, 6,201,639; 6,218,803; and 6,225,625, and in WO99/47964.

The practice of the present invention may also employ conventional biology methods, software and systems. Computer software products typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., Introduction to Computational Biology Methods (PWS Publishing Company, Boston, 1997); Salzberg, Searles, Kasif, (Ed.) Computational Methods in Molecular Biology, (Elsevier, Amsterdam, 1998; Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001). See U.S. Patent No. 6,420,108.

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Light patterns can also be generated using Digital Micromirrors, Light Crystal on Silicon (LCOS), light valve arrays, laser beam patterns and other devices suitable for direct-write photolithography. See, e.g., U.S. Patent Nos. 6,271,957 and 6,480,324.

Additionally, methods for providing genetic information over networks such as the Internet as shown in United States Publication No.US20020183936.

The present invention provides methods for the formation of arrays of large numbers of different polymer sequences. The methods and compositions described herein may involve the conversion of radiation signals into chemical products that are particularly useful in
polymer synthesis. Arrays formed using the methods of the invention are disclosed herein. There are also described herein methods, compositions, and devices for the synthesis of an array of different polymers in selected and predefined regions of a substrate. There are also described herein those arrays and various methods of using them.

Such arrays are used in, for example, in nucleic acid analysis. Polynucleotide or nucleic acid arrays are especially suitable for checking the accuracy of previously elucidated sequences and for detecting mutations and polymorphisms. Polymer arrays are also used in screening studies to evaluate their interaction with, for example, receptors such as antibodies in the case of peptide arrays or with nucleic acids in the case, for example of oligonucleotide arrays. For example, the screening of peptides to determine which if any of a diverse set of peptides has strong binding affinity with a receptor.

The arrays formed by the methods of the present invention can be used in competitive assays or other well-known techniques to screen for compounds having certain activities. For example, vast collections of synthetic or natural compounds are immobilized on predefined regions of a substrate. The reaction of the immobilized compounds (or compound) with various test compositions such as the members of a chemical library or a biological extract are tested by dispensing small aliquots of each member of the library or extract to a different region. In one instance, a large collection of human receptors is deposited on a substrate, one in each region to form an array. A plant or animal extract is then screened for binding to various receptors of the array.

Nucleic acid sequences can also be immobilized in specific locations or predefined regions of a substrate using the current invention. In some instances, such immobilized nucleic acid arrays are used in hybridization assays for gene expression monitoring, nucleic acid amplifications, nucleic acid computation, and nucleic acid analysis in general.

The present invention has certain features in common with the radiation directed methods discussed in U.S. Patent No. 5,143,854. The radiation-directed methods discussed in that patent involve activating predefined regions of the substrate and then contacting the substrate with a
preselected monomer solution. The predefined regions can be activated with, for example, a light source shown through a mask (much in the manner of photolithographic techniques used in integrated circuit fabrication). Other regions of the substrate remain inactive because they are blocked by the mask from illumination. Thus, a light pattern defines which regions of the substrate react with a given monomer. By repeatedly activating different sets of predefined regions and providing different monomer compositions thereto, a diverse array of polymers is produced on or near the substrate.

According to one embodiment of the present invention, linker molecules having reactive functional groups protected by protecting groups are provided on the surface of a substrate. In one preferred instance a catalyst system including a photoacid generator ("PAG") and a base, but no sensitizer, are provided on the surface, preferably in a film. In accordance with the present invention, the catalyst system comprises a film comprising a PAG, a sensitizer and a base. A set of selected regions on the surface of the substrate is exposed to radiation using well-known lithographic methods discussed, for example, in Thompson, L.F.; Willson, C.G.; and Bowden, M.J., Introduction to Microlithography; American Chemical Society, 1994, pp. 212-232. The generated acid is allowed to be exposed to the protected group for long enough and under sufficient conditions to remove the protective group, preferably a DMT group. Afterwards, the surface of the array is stripped, preferably in an appropriate solvent leaving protected and unprotected groups. Monomers having a protective group are allowed to react with the exposed groups. The surface is again coated with one of the catalyst systems described above. A second set of selected regions is exposed to radiation as above.

A second set of selected regions is, thereafter, exposed to radiation. The radiation-initiated reactions remove the protecting groups on molecules in the second set of selected regions, i.e., the linker molecules and the first-bound monomers. The substrate is then contacted with a second monomer containing a removable protective group for reaction with exposed functional groups. This process is repeated to selectively apply monomers until polymers of a desired length and desired chemical sequence are obtained. According to one aspect of the present
invention, the growing chains of nucleic acid can be capped in between synthesis rounds. This procedure limits the production of nucleic acids with an undesired sequence. Side chain protective groups for exocylic amines for example, if present, are also optionally removed.

In one preferred embodiment, the monomer is a 2'-deoxynucleoside phosphoramidite containing an acid removable protecting group at its 5'hydroxyl group. As stated previously, in an alternate embodiment, the protecting group is present at the 3' hydroxyl group if synthesis of the polynucleotide is from the 5' to 3' direction. The nucleoside phosphoroamidite is represented in accordance with one aspect of the present invention by the following formula: 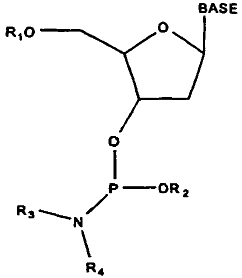 wherein the base is adenine, guanine. thymine, or cytosine, R₁ is a protecting group which makes the 5' hydroxyl group unavailable for reaction and includes dimethoxytrityl, tert-butyloxycarbonyl or any of the protecting groups known to those of skill in the art; R₂ is cyanoethyl, methyl, t-butyl, trimethylsilyl and the like and R₃ and R₄ are isopropyl, cyclohexane and the like. Exocyclic amines present on the bases can also be protected with acyl protecting groups such as benzoyl, isobutyryl, phenoxyacetyl and the like. The linker molecule contains an acid- or base- removable protecting group. Useful linker molecules are well known to those skilled in the art and representative examples include oligo ethers such as hexaethylene glycol, oligomers of nucleotides, esters, carbonates, amides and the like. Useful protecting groups include those previously listed and others known to those skilled in the art.

In another preferred embodiment, the monomer is an amino acid containing an acid- or base- removable protecting group at its amino or carboxy terminus and the linker molecule terminates in an amino or carboxy acid group bearing an acid- or base-removable protecting group. Protecting groups include tert-butyloxycarbonyl, 9-fluorophenylmethoxycarbonyl, and any of the protective groups previously mentioned and others known to those skilled in the art.

According to one aspect of the present invention, spatially defined polymer synthesis will be performed by depositing a photoresist such as Ghand's "VLSI Fabrication Principles," Wiley (1983). According to these embodiments, a resist is deposited, selectively exposed, leaving a portion of the substrate exposed for coupling. These steps of depositing resist, selectively removing resist and monomer coupling are repeated to form polymers of defined sequences at desired locations. In some specific embodiments, a positive tone resist comprised of diazonapthoquinone-novolac (DQN/N) is incorporated in a creasole-formaldehyde polymer matrix. This resist and its variants are used routinely in the microelectronics industry for submicron resolution lithography, as more fully discussed in Reiser, "Photoreactive Polymers: The Science and Technology of Resist," Riley (1989). However, it has been discovered in accordance with an aspect of the present invention that substantial and non-obvious refinements to the procedures developed for the microelectronics industry are necessary to allow similar procedures to work with certain polymers of the present invention, e.g., nucleic acids. It is also known to those of skill in the art that other polymers such as peptides are not stable at all conditions employed in the microelectronics industry.

High contrast detritylation of < 4 microns has been demonstrated with simple contact printing with a resist. Unfortunately, the alkaline conditions needed (aqueous [OH] 0.1 M) complicates its direct use in a multistep polymer synthesis, such as polynucleotide array fabrication because of the hydrolysis of nucleobase exocylic amine protecting groups that are used to prevent side reactions during synthesis with standard phosphoramidite monomers.

As various well known methods for chemical removal of DMT protecting groups involving application of alkali conditions resulted in undesired side reactions such as removal of exo-cyclic amino protecting groups, reagents and methods were developed for light-directed synthesis of DNA probes, utilizing phosphoramidite monomers having photolabile protecting groups. These methods and reagents are described in the various references.

Under some circumstances, photodeprotection yields truncated probe sequences due to incomplete removal of the photoprotecting group following application of light. Incomplete removal of a photodeprotecting group may impose limitations on probe length. For example, if one imagines a stepwise yield of photolysis of 85% and 25 successive steps are carried out to provide 25-mer oligonucleotides, less than 2% of the probes will reach the desired length of 25.

In addition, relative to conventional DMT-protected phosphoramidite monomers, photolabile-protected phosphoramidite monomers are costly to obtain. A manufacturing process that uses DMT-protected phosphoramidite monomers should therefore be cheaper, and by analogy to well-established efficiencies of acid-mediated DMT removal, should also be higher-yielding, perhaps even approaching a 99% stepwise yield. A high-yielding synthesis method would substantially decrease the number of truncated probes and enable the ability to produce long-mer probes (e.g., 50-mer, 60-mer, 70-mer etc.) with relative ease. Shorter probes could also be constructed by the same method if desired.

In accordance with one aspect of the present invention, methods to generate localized photo-generation of appropriate acid species to effect DMT removal from growing strands of polynucleotides were developed. The traditional semiconductor field employs photoacid generator compounds (i.e., PAGs) in conjunction with "sensitizer" compounds that require elevated temperatures to achieve a suitable acidity to appropriately affect surfaces in that industry. In accordance with an aspect of the present invention, it was discovered that some polymers, for example polynucleotides, including DNA, are susceptible to depurination at elevated temperatures and low pH values, giving rise to variably degraded probes. Probes which have undergone depurination, i.e., the loss of the base structure on T and C nucleotides, will not hybridize as well to corresponding homologous DNA or RNA. Substantially damaged probes may not hybridize at all or may hybridize without specificity, i.e., background hybridization unrelated to sequence of probe. Arrays with a substantial number of depurinated probes would be undesirable for a number of reasons including possible failure to hybridize to theoretically homologous nucleic acids in a sample, resulting in a false negative experiment. Solutions to acid induced depurination are known in the art. Analogues of standard DNA, for example 2'-OMe nucleoside modifications, are known to be more resistant to such degradation. However, utilization of such analogues is substantially more expensive than the corresponding underivatized analog. Moreover, analogues such as 2'-OMe nucleosides alter the hybridization properties of the probes, which would require changes to probe/array design.

It has been discovered in accordance with the present invention that high-yield probes may be prepared using standard DMT-containing monomers and detritylation with a photoacid generator used under appropriate conditions, i.e., conditions described in accordance with an aspect of the present invention which substantially reduce or eliminate acid induced depurination. In accordance with an aspect of the present invention, the local concentration of acid liberated by the activated PAG, which is reflected by the pKa of this molecule, is an important consideration in selecting the appropriate PAG(s) for the particular polymer to be fabricated. Also, the exposure time of the polymer to the acid is another important consideration. Another key aspect of an aspect of the invention is the photolysis time, which must be of sufficient duration to generate a suitable quantity of acid and achieve essentially quantitative detritylation, but not so long that depurination becomes a factor. It has been discovered in accordance with an aspect of the present invention that a heating step following photoactivation of the PAG, which is routinely employed and taught in the semiconductor industry, should not be used in conjunction with certain polymers contemplated by the present invention, including especially polynucleotides, e.g., DNA oligonucleotides. If growing polynucleotide chains are baked after activation of the photoacid generator, it appears that the resulting heat in conjunction with a localized low pH causes depurination. Thus, post-UV light exposure baking is to be avoided in accordance with an aspect of the present invention.

With respect to on particular aspect of the present invention, it has been discovered that certain onium salts provide excellent removal of the DMT group when used in conjunction with an appropriate base and without a post-exposure baking step. In another aspect of the present invention, a non-ionic PAG is used in conjunction with a sensitizer and a base to provide high yield DMT removal without causing unwanted depurination. These approaches, in accordance with an aspect of the present invention, substantially solve the problem of probe degradation often observed with photoacid generation, avoid the need to use DNA analogues and enables a high-yield probe synthesis process and resulting products.

In accordance with this aspect of the present invention, the photoacid causes minimal or insubstantial damage to the polymers making up the array. What damage may be endured by the polymer in question will be determined by the nature of the polymer and the assay or experiment to be conducted with the array. This will be apparent to the person of skill in the art. For example, if an array of oligonucleotides is fabricated, a certain amount of depurination may be tolerated if the probes on the array can still be used to reliably and specifically detect sequences in a sample.

In accordance with another aspect of the present invention, the PAG must be chosen such that that wave length of light of activation is not too short. For example, many PAGs are used in the semiconductor industry which require UV light have a wavelength of less than 300nm. Indeed, literature references speak of using "short UV" PAGs wherein wavelengths of light of 220 to 260 nm are used. In accordance with an aspect of the present invention, such short UV wavelengths are totally unacceptable with respect to certain polymers, particularly nucleic acids. For nucleic acids UV light is used on the order of preferably 330 to 365 nm. More preferably, UV light of around 365 nm is used.

A process is described herein for fabricating an array of polymers, the process having the steps of providing a solid substrate having a reactive group protected by a protective group; coating the solid substrate with a film having an activatable deprotecting agent; activating the deprotecting agent in selected areas by selective application of an applicator to provide an activated deprotecting agent; and exposing the monomer having the protective group to the activated deprotecting group under appropriate conditions such that the protecting group is removed to provide an exposed reactive group wherein the step of exposing does not result in substantial damage to the polymer. In accordance with the present invention the reactive group may be located on a linker having one end bound to a solid substrate with the reactive group at the opposite end or other exposed site of the linker, a monomer attached to a linker or a polymer (here two or more monomers) attached to a linker.

Preferably the array of polymers is an array of nucleic acids. More preferably, the array of nucleic acids is an array of oligonucleotides. The monomer is preferably a naturally or non-naturally occurring nucleotide. More preferably the nucleotide is selected from the group consisting of G, A, T, and C. Preferably, the nucleotide is protected at its 5' hydroxyl end by a dimethoxytrityl ("DMT") protective group. In the most preferred embodiments, the nucleotide is selected from the group G, A, T, and C and is protected at its 5' hydroxyl group by a DMT protective group. In another aspect of the present invention, the nucleotide is protected at its 3' hydroxyl group with a DMT protective group. Thus, in accordance with the present invention, nucleotides may be synthesized in the 5' to 3' direction or a 3' to 5' direction. In still another preferred embodiment of the present invention, the array of polymers is an array of peptides. Also, preferably, the monomer is an amino acid. It is also a preferred embodiment of the present invention that the amino acid is a naturally occurring amino acid or a non-naturally occurring amino acid. More preferably the amino acid is selected from the group consisting of alanine, arginine, asparagine, aspartic acid, cysteine, glutamine, glutamic acid, glycine, histidine, isoleucine, leucine, lysine, methionine, phenylalanine, praline, serine, threonine, tryptophan, tyrosine and valine.

In still another preferred embodiment, the amino acid is protected at its amino terminus functionality by a tert-butyloxycarbonyl ("tBOC") protective group during synthesis.

In another aspect of the instant invention, the process described above has an additional step of reacting the monomer with an exposed reactive group with a second monomer having a reactive group protected by a protective group. In another preferred embodiment of the instant invention, the process has a further step of repeating all the steps to obtain the desired polymer array.

Originally the term lithography referred to a method of printing using a nonpolar ink applied to a hydrophilic master plate patterned with a hydrophobic image. As used at the present date, the term is generally used to describe a number of methods for replicating a predetermined master pattern on a substrate. Common applications of this technology involve replication effected by first coating the substrate with a radiation-sensitive polymer film (a resist) and then exposing the film to actinic radiation in a predefined pattern. The radiation induced chemical changes that result, alter the chemical properties of the exposed regions of the coated substrate such that they can be differentiated in subsequent developmental steps.

In yet another preferred embodiment of the instant invention, the step of coating is performed by applying to the substrate a film of a polymer solution containing the activatable deprotecting agent. Preferably, the polymer solution is a composition of a certain percentage of poly(methyl methacrylate). Preferably, the activatable deprotecting agent is a photoacid generator. Both ionic and non-ionic photoacid generators can be used in accordance with an aspect of the present invention. Preferably the photoacid generator is 2,6-dinitrobenzyl tosylate, a non-ionic photoacid generator. Where the activatable deprotecting agent is a photoacid generator, it is particularly preferred that the monomer is a nucleotide and the protecting group is DMT. It is also preferred in this situation that the monomer is an amino acid and the protecting group is tBOC.

Where the activatable deprotecting agent is a photoacid and the photoacid is 2,6-dinitro benzyl tosylate, the activator is preferably light having a wave length of around 365 nm. In still other preferred embodiments of the instant invention, the array of polymers comprises a polymer at least 50 monomers in length. In other preferred embodiments, the polymer is at least 60 monomers in length. In still other preferred embodiments, the polymer is at least 70 monomers in length. More preferably, each of the at least 50, 60 and 70 monomer long polymers are DNA oligonucleotides.

Still other photoacid generators ("PAGs") are known. Common commercial ionic PAGs include onium and organometallic salts such as diaryliodonium and triarylsulfonium salts and (cyclopentadienyl)(arene)iron⁺ salts of the anions PF₆⁻, SbF₆⁻, CF₃SO₃⁻, C₄F₉SO₃⁻ and C₈F₁₇SO₃⁻. Also known are sulfonium salts (e.g., triphenylsulfonium hexafluorophosphate, triflate, toslyate, and camphorsulfonate The photochemical reaction of many onium salt generates a low concentration of a strong Brönsted acid. In this regard, numerous PAGs are known from the semiconductor industry. However, in the semi-conductor industry, the wafer is subjected to a baking step after generation of the acid by photolysis, where the exposed wafers are subjected to temperatures exceeding 100°C for prolonged periods of time. In accordance with the present invention, it has been discovered that baking has a deleterious effect on some polymers, in particular nucleic acids. Thus, while onium salts and other PAGs used in the semiconductor industry are of interest to the present invention, protocols for the usage of these compounds must be varied significantly as described in accordance with the present invention.

Onium salts are known to have high quantum yields of acid production, good absorption properties and good solubility in many resist films. However, it is also known in accordance with the present invention that the wavelengths of light commonly used to activate onium salts for semi-conductors can not be used with some polymers, particularly nucleic acids. In this regard, it is common in the semi-conductor industry to use low wavelength UV light (e.g. less than 300 nm) to activate onium salts. See, e.g., Wallraff, G.M. and Hinsberg, W.D., Lithographic Imaging Techniques for the formation of Nanoscopic Features, Chem. Rev. 1999, 99, 1801-1821.

In accordance with the present invention, it is known that such wavelengths of light are entirely unacceptable for the synthesis of nucleic acids. Such wavelengths of UV light cause numerous forms of damage to a nucleic acid chain, including crosslinking of bases. Nucleic acids synthesized under these conditions would be unable to hybridize to their homologous counterparts. To use onium salts in accordance with the present invention, they must absorb light in the range of 330 nm to about 365 nm and generate acid at an acceptable level and rate (photospeed) at those longer wavelengths. Such onium salts are known in the literature or could be devised based on the teachings of present invention by those of skill in the art using reasonable and not undue effort.

Many onium salts can be synthesized by metathesis reactions. Thus, the acid counterion can be easily modified. In turn, this allows a ready means to vary the pKa, volatility and size of the photogenerated acid. Onium acids are described in a wide variety of published references, including Wallraff, G.M. and Hinsberg, W.D., cited above. See also Shirai, M and Tsunooka, M., "Photoacid and Photobase Generators: Chemistry and Applications to Polymeric Materials," Prov. Polym. Sci., Vol. 21, 1-45, 1996.

In accordance with an aspect of the present invention, both ionic and non-ionic photoacid generators are contemplated. Both have advantages and disadvantages. Ionic PAGs are thermally stable and have a wide range of spectral absorption. However, ionic solvents have a limited solubility in organic solvents. Non-ionic PAGs have a better solubility in organic solvents, but have less thermal stability than ionic PAGs. However, as discussed above, the thermal stability is less of an important consideration for the present invention.

In accordance with an aspect of the present invention, it is important that the polymers to be synthesized by the techniques of the present invention not undergo undue or substantial damage during the synthesis. In this regard, it is known that exposure of nucleic acid polymers to acids can result in damage, including for example depurination. In the context of nucleic acid microarrays, which are used to detect the hybridization of homologous species of nucleotides, the nucleic acid attached to the substrate can undergo some depuriation and still act to satisfactorily hybridize homologous nucleic acids. However, if the damage is too great, the hybridization will not occur at all or will not occur reliably. A substantial number of damaged proves in a feature could result in a false negative. Thus, it is important in embodiments of the instant invention employing photoacid generators that the acid is not allowed to substantially damage the nucleic acids being synthesized. In accordance with the present invention, substantial damage means that the polymer or nucleic acid is unable to be used for the intended use for the array. Thus, in the context of a nucleic acid array, substantial damage would mean that the array could not be used to reliably detect nucleic acids. For a protein array, substantial damage would mean that the peptide was damaged to the extent that it could not be recognized by an antibody or protein receptor.

A process for fabricating an array of polymers is described herein, the method having the steps of providing a solid substrate comprising a monomer having a reactive group protected by a protective group; coating the solid substrate with a film, said film comprising an activatable deprotecting agent; activating the deprotecting agent in selected areas by selective application of an activator to provide an activated deprotecting agent; exposing the monomer having the protective group to the activated deprotecting group under appropriate conditions such that the protecting group is removed to provide a monomer with an exposed reactive group wherein the step of exposure does not result in substantial damage to the polymer.

The array is preferably an array of nucleic acids or an array of oligonucleotides. The monomer is preferably a nucleotide. More preferably the nucleotide is protected at its 5' hydroxyl end with a DMT protective group. It is also preferred that the nucleotide is protected at its 3' hydroxyl group with a DMT protective group.

In still other preferred embodiments of the present invention the polymer is a peptide. The monomer is preferably an amino acid. More preferably, the amino acid is a naturally occurring amino acid. In still other embodiments of the instant invention, it is preferred that the amino acid is protected at its amino functionality by a tBOC protective group.

In other preferred embodiments the nucleotide, is selected from the group consisting of G, A, T and C. More preferably, the nucleotide selected from the group consisting of G, A, T, and C is protected at its 5' hydroxyl group with a DMT protective group.

In an other preferred embodiment of the instant invention, the process comprises the further step of reacting said exposed reactive group with a monomer having a reactive group protected by a protective group; coating the solid substrate with a film having an activatable deprotecting agent, activating said deprotecting agent in selected areas by selective application of an activator to provide an activated deprotecting agent; exposing the monomer having the protective group to said activated deprotecting group under appropriate conditions such that said protecting group is removed to provide a monomer with an exposed reactive group wherein said step of exposure does not result in substantial damage to said polymer and repeating the above steps to provide the desired polymer array.

In another preferred embodiment of the present invention the step of coating is performed by applying to the substrate a film of a polymer solution containing said activatable deprotecting agent. Preferably, the activatable deprotecting agent is a photoacid generator. More preferably the photoacid generator is selected from the group consisting of a photoacid generator selected from the group consisting of an ionic photoacid generator and a non-ionic generator. In another preferred embodiment, the photoacid generator is 2,6-dinitrobenzyl tosylate. Where the activatable deprotecting agent is a photoacid generator the monomer comprises a nucleotide and the protecting group is DMT. In another preferred embodiment of the present invention, the monomer is an amino acid and the protecting group is tBOC. Where a photoacid generator is used, it is preferably dispersed in poly(methyl methacrylate) (PMMA).

Where the monomer is a nucleic acid, the activator is preferably light having a wave length of between 330 and 365 nm. It is also a preferred embodiment of the present invention that the array of polymers comprises a polymer at least 25 to 75 monomers in length.

In a preferred embodiment of the present invention the photoacid generator is an onium salt. More preferably, the onium salt is Bis (4-t-butyl phenyl) iodonium PF₆⁻.

In one preferred embodiment of the present invention, where the polymer is a nucleic acid, substantial damage is determined by the ability of the nucleic acid array to bind complementary nucleic acids.

In accordance with the present invention after exposing the photoacid generator to an activating wavelength of light, there is no post exposure baking or heating step.

In preferred embodiments of the present invention, the polymer is a nucleic acid and the monomer is a nucleotide and substantial damage is determined by determining the level of false negatives generated by hybridizing the array with a known sample having known complementary nucleic acids to said array. In accordance with this aspect of the present invention, the array could be tested by hybridizing it with a test or control sample having nucleic acids which should give a positive signal on the array if the oligonucleotides, for example, on the array have been synthesized without substantial damage. After hybridization of the control sequence, the array can be scanned and the features analyzed with the corresponding control probes. If the control probes have suffered no damage during fabrication, a high intensity result should be observed. However, if minimal damage occurred the signal might still be present, but diminished, for example by 50%. If the array were intended to detect rare species such a diminution would probably not be acceptable. The batch of arrays containing such defects would likely have to be disposed of. If no signal were seen or if the signal was diminished by 90% or more, the batch of such arrays would probably have to be disposed of regardless of the proposed end use of such arrays

In accordance with another aspect of the present invention, an array of oligonucleotides is produced using a PAG and DMT protected nucleotides to produce features preferably on the order of 10-100 µm. More preferably, features are on the order 1-10 µm. In another preferred embodiment, features are on the order of 100-1000 nm.

### EXAMPLES

### Example 1: 2,6-dinitrobenzyl tosylate PAG

In this example, a PAG was used in conjunction with standard DMT-protected phosphoramidite monomers to fabricate oligonucleotide arrays. A solution of activated DMT-protected phosphoramidite monomer was coupled to a support-bound hydroxyl functionality and oxidized in the typical manner. The support (i.e., wafer or chip) was removed from the flowcell and coated with a polymer solution that contained a photoacid generator: 2,6-dinitrobenzyl tosylate ("DBT").

A film was prepared of 10% by weight DBT incorporated in 15% PMMA (MW 120k) in MEK solvent, including a base of 0.5% di-t-butyl aniline and spun coat at 2,500 RPM for 90 seconds onto the substrate, which is a convenient method to apply the polymer solution, and provides a tact-free surface. The coated support was then subjected to photolysis with (or without a mask for certain control experiments) using a dosage of about 1 Joule at 365 nm wavelength. Following photolysis, the support was promptly stripped of its coating by applying with acetonitrile, and then the support was returned to the flowcell to continue probe synthesis. This basic sequence of events was repeated to add additional monomer units, thus assembling the probe. After the desired probes had been synthesized, the substrate was base-deprotected in the normal way and then used in hybridization experiments.

Supporting data demonstrate exemplary methods described above in accordance with one aspect of the present invention. 20-mer and 50-mer probes were prepared in various patterns. Hybridization signals and profiles from these were compared to a "gold standard" method using solution-phase TCA delivery to achieve detritylation. In most respects, the behavior of the probes prepared with the photoacid generator process is identical to the behavior of the probes prepared with conventional solution-phase TCA detritylation. This observation demonstrates that the stepwise coupling yield for the probes prepared by the photo-acid generator process is comparable to that achieved with solution-phase TCA delivery (i.e., 97-99%). Moreover, the hybridization results further demonstrate that the probe is intact and not degraded as a result of depurination and subsequent chain cleavage. Particularly low background signal was obtained. The low levels of background demonstrate that this method additionally holds promise for array designs that demand extremely high-contrast, such as those that contain ultra-small features. No baking step was conducted between the photolysis step and the stripping step in the above process.

### Example 2: Onium Salt PAG

In yet another aspect of the present invention, an onium salt was used as a photoacid generator. Bis (4-t-butyl phenyl) iodonium PF₆⁻ (5% wt., 80 mM) was used in a polymer of 5% (wt) PMMA (15k) in ethyl lactate. Also included in the formulation was a sensitizer and a base. The sensitizer was 2-isopropyl thioxanthone (ITX, 9.5%wt., 371mM) and the base N-octylamine (0.85% wt., 65.8mM). ITX has the following structure(s):

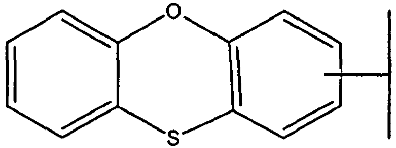

The polymer and formulation PAG, sensitizer and base was spun coat for 60 seconds at 3000 RPM on to a substrate to generate a layer of 0.1 µm thickness, followed by a prebake for 1 minute at 85 degrees centigrade.

Exposure of the spun coated, prebaked plate was at 66 mJ for non-base formulation and 120 mJ for base-added formulation. Following exposure, stripping was performed with SVC-14 (60% DMSO: 40% aliphatic ether), ACN. Hybridization to 10 nM target in 1XMES at 35 degrees C. No post baking step was performed. Scanning was performed using an Agilent instrument at 530 nm (3µm), an ARC instrument at 570 nm (1 µm), and by SEM.

Synthesis fidelity of the onium system was analyzed. The hexamer 3'-TAGCAT-5' was fabricated with the constituents as identified above. The total yield was 64% and the stepwise yield was 94%. The lithographic performance was also analyzed and the onium photoresist provided high contrast arrays with excellent resolution. The onium process is also robust as was demonstrated by a 75-step wafer scale synthesis. Because of the high total and stepwise yield, the onium photoacid generator can be used to generate arrays with longer oligonucleotide probes than currently available photolithographic methods. In this regard, the onium system described above was used to synthesize 50 mer probes. High intensity signals were sign on hybridization to these 50 mers. Moreover, little depurination was observed.

The feature size of onium arrays produced with different masks was measured and is shown in Table 1 below:

**Table 1 (20/20 oligo213 hyb SEM observation)**

| Mask Feature (µm) | Actual Feature Observed (µm) | Bias (%) |
|---|---|---|
| 3.0 | 3.3 | 10 |
| 2.5 | 2.8 | 12 |
| 2.0 | 2.2 | 13 |
| 1.5 | 1.7 | 13 |

In summary, the onium based salt supporting data demonstrate exemplary methods described above in accordance with one aspect of the present invention. 20-mer and 50-mer probes were prepared in various patterns. Hybridization signals and profiles from these were compared to a "gold standard" method using solution-phase TCA delivery to achieve detritylation. In most respects, the behavior of the probes prepared with the photoacid generator process is identical to the behavior of the probes prepared with conventional solution-phase TCA detritylation. This observation demonstrates that the stepwise coupling yield for the probes prepared by the photo-acid generator process is comparable to that achieved with solution-phase TCA delivery (i.e., 97-99%). Moreover, the hybridization results further demonstrate that the probe is intact and not degraded as a result of depurination and subsequent chain cleavage. Particularly low background signal was obtained. The low levels of background demonstrate that this method additionally holds promise for array designs that demand extremely high-contrast, such as those that contain ultra-small features. No baking step was conducted between the photolysis step and the stripping step in the above process.

The foregoing invention has been described in some detail by way of illustration and examples, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims..

## Claims

1. A process for fabricating an array of polymers comprising:
providing a solid substrate comprising a reactive group protected by a protective group;
coating said solid substrate with a film, said film comprising a photoacid generator, a sensitizer and a base, wherein the film is spun coated onto the substrate;
activating said photoacid generator in selected areas by selective application of light to produce an acid wherein a step of baking is not performed following activation of the photoacid generator; and
exposing said reactive group having said protective group to said acid under appropriate conditions such that said protecting group is removed to provide a monomer with an exposed reactive group wherein said step of exposure does not result in substantial damage to said polymers of the array.

2. A process according to claim 1 wherein said array of polymers comprises an array of nucleic acids.

3. A process according to claim 2 wherein said array of nucleic acids comprises an array of oligonucleotides.

4. A process according to claim 1 wherein said reactive group comprises a molecule selected from the group consisting of a linker, a monomer, and a polymer.

5. A process according to claim 4 wherein said molecule is a monomer comprising a nucleotide.

6. A process according to claim 5 wherein said nucleotide is protected at its 5' hydroxyl end with a DMT protective group.

7. A process according to claim 5 wherein said nucleotide is protected at its 3' hydroxyl group with a DMT protective group.

8. A process according to claim 5 wherein said nucleotide is selected from the group consisting of G, A, T and C.

9. A process according to claim 8 wherein said nucleotide selected from the group consisting of G, A, T, and C is protected at its 5' hydroxyl group with a DMT protective group.

10. A process according to claim 4 wherein said molecule is a monomer comprising an amino acid.

11. A process according to claim 10 wherein said amino acid is a naturally occurring amino acid or a non-naturally occurring amino acid.

12. A process according to claim 10 wherein the amino acid is selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

13. A process according to claim 10 wherein said amino acid is protected at its amino functionality by a tBOC protective group.

14. A process according to any of claims 1 to 13 wherein said light has a wave length of between 330 and 365 nm.

15. A process according to claim 4 wherein said polymer is a nucleic acid and said monomer is a nucleotide and substantial damage is determined by determining the level of false negatives generated by hybridizing the array with a sample having known complementary nucleic acids to said array.

16. A process according to claim 1 wherein said polymer is a peptide.

17. A process according to claim 1 further comprising the steps of
stripping the film from the substrate with an appropriate solvent after removal of the protective group to provide a partially completed substrate comprising a monomer with an exposed reactive group;
reacting said monomer with an exposed reactive group with a second monomer having a reactive group protected by a protective group; and
repeating the steps of coating, activating, exposing, stripping, and reacting to provide the desired polymer array.

18. A process according to claim 17 wherein said reactive group comprises a nucleotide with a DMT protective group.

19. A process according to claim 18 wherein said photoacid generator is selected from the group consisting of an ionic photoacid generator and a non-ionic generator.

20. A process according to claim 19 wherein said photoacid generator is an ionic photoacid generator.

21. A process according to claim 20 wherein said ionic photoacid generator is an onium salt.

22. A process according to claim 21 wherein said onium salt is Bis (4-t-butyl phenyl) iodonium PF₆⁻.

23. A process according to claim 19 wherein said photoacid generator is a non-ionic photoacid generator.

24. A process according to claim 23 wherein said non-ionic photoacid generator is 2,6-dinitrobenzyl tosylate.

25. A process according to claim 1 wherein the film comprises poly(methyl methacrylate).

26. A process according to claim 1 wherein said photoacid generator is an onium salt.

27. A process according to claim 26 wherein said onium salt is Bis (4-t-butyl phenyl) iodonium PF₆⁻.

28. A process according to claim 27 wherein said sensitizer is 2-isopropyl thioxanthone.

29. A process according to claim 1 wherein said array of polymers comprises a polymer at least 50 monomers in length.

30. A process according to claim 29 wherein said array of polymers comprises a polymer at least 60 monomers in length.

31. A process according to claim 30 wherein said array of polymers comprises a polymer at least 70 nucleotides in length.

32. A process according to claim 31 wherein said polymer is a DNA oligonucleotide.

33. A process according to claim 30 wherein said polymer is a DNA oligonucleotide.

34. A process according to claim 29 wherein said polymer is a DNA oligonucleotide.

## Patentansprüche

1. Verfahren zur Herstellung einer Anordnung von Polymeren, das Folgendes aufweist:
Bereitstellen eines Festsubstrats, das eine reaktive Gruppe aufweist, die von einer Schutzgruppe geschützt ist;
Beschichten des Festsubstrats mit einem Film, wobei der Film einen Photosäure-Erzeuger, einen Sensibilisator und eine Basis aufweist, wobei der Film auf das Substrat aufgeschleudert wird;
Aktivieren des Photosäure-Erzeugers in ausgewählten Bereichen durch selektives Aufbringen von Licht zur Erzeugung einer Säure, wobei ein Schritt des Brennens nicht anschließend an die Aktivierung des Photosäure-Erzeugers durchgeführt wird; und
Freilegen der reaktiven Gruppe, die die Schutzgruppe aufweist, für die Säure unter geeigneten Bedingungen, so dass die Schutzgruppe entfernt wird, um ein Monomer mit einer freiliegenden reaktiven Gruppe bereitzustellen, wobei der Schritt des Freilegens nicht zu erheblichem Schaden an den Polymeren der Anordnung führt.

2. Verfahren nach Anspruch 1, wobei die Anordnung von Polymeren eine Anordnung von Nucleinsäuren aufweist.

3. Verfahren nach Anspruch 2, wobei die Anordnung von Nucleinsäuren eine Anordnung von Oligonucleotiden aufweist.

4. Verfahren nach Anspruch 1, wobei die reaktive Gruppe ein Molekül aufweist, das aus der Gruppe bestehend aus einem Binder, einem Monomer und einem Polymer ausgewählt ist.

5. Verfahren nach Anspruch 4, wobei das Molekül ein Monomer ist, das ein Nucleotid aufweist.

6. Verfahren nach Anspruch 5, wobei das Nucleotid an seinem 5'-Hydoxylende von einer DMT-Schutzgruppe geschützt ist.

7. Verfahren nach Anspruch 5, wobei das Nucleotid an seiner 3'-Hydroxylgruppe von einer DMT-Schutzgruppe geschützt ist.

8. Verfahren nach Anspruch 5, wobei das Nucleotid aus der Gruppe bestehend aus G, A, T und C ausgewählt ist.

9. Verfahren nach Anspruch 8, wobei das Nucleotid, das aus der Gruppe bestehend aus G, A, T und C ausgewählt ist, an seiner 5'-Hydroxylgruppe von einer DMT-Schutzgruppe geschützt ist.

10. Verfahren nach Anspruch 4, wobei das Molekül ein Monomer ist, das eine Aminosäure aufweist.

11. Verfahren nach Anspruch 10, wobei die Aminosäure eine natürlich auftretende Aminosäure oder eine nicht-natürlich auftretende Aminosäure ist.

12. Verfahren nach Anspruch 10, wobei die Aminosäure aus der Gruppe bestehend aus Alanin, Arginin, Asparagin, Aspartinsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin ausgewählt ist.

13. Verfahren nach Anspruch 10, wobei die Aminosäure an ihrer Amino-Funktionalität von einer tBOC-Schutzgruppe geschützt ist.

14. Verfahren nach einem der Ansprüche 1 bis 13, wobei das Licht eine Wellenlänge von zwischen 330 und 365 nm aufweist.

15. Verfahren nach Anspruch 4, wobei das Polymer eine Nucleinsäure ist und das Monomer ein Nucleotid ist, und wesentlicher Schaden durch Bestimmen des Grads an falschen Negativen bestimmt wird, die durch Hybridisieren der Anordnung mit einer Probe mit für die Anordnung bekannten komplementären Nucleinsäuren erzeugt werden.

16. Verfahren nach Anspruch 1, wobei das Polymer ein Peptid ist.

17. Verfahren nach Anspruch 1, das ferner die folgenden Schritte aufweist:
Abziehen des Films von dem Substrat mit einem geeigneten Lösungsmittel nach der Entfernung der Schutzgruppe zur Bereitstellung eines teilweise vervollständigten Substrats, das ein Monomer mit einer freigelegten reaktiven Gruppe aufweist;
Bringen des Monomers zur Reaktion mit einer freigelegten reaktiven Gruppe mit einem zweiten Monomer, das eine reaktive Gruppe aufweist, die von einer Schutzgruppe geschützt ist; und
Wiederholen der Schritte des Beschichtens, Aktivierens, Freilegens, Abziehens und Reagierens zur Bereitstellung der erwünschten Polymeranordnung.

18. Verfahren nach Anspruch 17, wobei die reaktive Gruppe ein Nucleotid mit einer DMT-Schutzgruppe aufweist.

19. Verfahren nach Anspruch 18, wobei der Photosäure-Erzeuger aus der Gruppe bestehend aus einem ionischen Photosäure-Erzeuger und einem nicht-ionischen Photosäure-Erzeuger ausgewählt ist.

20. Verfahren nach Anspruch 19, wobei der Photosäure-Erzeuger ein ionischer Photosäure-Erzeuger ist.

21. Verfahren nach Anspruch 20, wobei der ionische Photosäure-Erzeuger ein Oniumsalz ist.

22. Verfahren nach Anspruch 21, wobei das Oniumsalz Bis(4-t-butylphenyl)iodonium PF₆⁻ ist.

23. Verfahren nach Anspruch 19, wobei der Photosäure-Erzeuger ein nicht-ionischer Photosäure-Erzeuger ist.

24. Verfahren nach Anspruch 23, wobei der nicht-ionische Photosäure-Erzeuger 2,6-Dinitrobenzyl-Tosylat ist.

25. Verfahren nach Anspruch 1, wobei der Film Poly(methylmethacrylat) aufweist.

26. Verfahren nach Anspruch 1, wobei der Photosäure-Erzeuger ein Oniumsalz ist.

27. Verfahren nach Anspruch 26, wobei das Oniumsalz Bis(4-t-butylphenyl)iodonium PF₆⁻ ist.

28. Verfahren nach Anspruch 27, wobei der Sensibilisator 2-Isopropylthioxanthon ist.

29. Verfahren nach Anspruch 1, wobei die Anordnung von Polymeren ein Polymer mit einer Länge von mindestens 50 Monomeren aufweist.

30. Verfahren nach Anspruch 29, wobei die Anordnung von Polymeren ein Polymer mit einer Länge von mindestens 60 Monomeren aufweist.

31. Verfahren nach Anspruch 30, wobei die Anordnung von Polymeren ein Polymer mit einer Länge von mindestens 70 Nucleotiden aufweist.

32. Verfahren nach Anspruch 31, wobei das Polymer ein DNA-Oligonucleotid ist.

33. Verfahren nach Anspruch 30, wobei das Polymer ein DNA-Oligonucleotid ist.

34. Verfahren nach Anspruch 29, wobei das Polymer ein DNA-Oligonucleotid ist.

## Revendications

1. Procédé de fabrication d'une matrice de polymères consistant à :
fournir un substrat solide comprenant un groupe réactif protégé par un groupe protecteur ;
revêtir le substrat solide avec un film, le film comprenant un générateur de photo acide, un sensibilisateur et une base, dans lequel le film est revêtu par filage sur le substrat ;
activer le générateur de photo acide dans des zones choisies par l'application sélective d'une lumière pour produire un acide, dans lequel une étape de cuisson n'est pas effectuée après l'activation du générateur de photo acide ; et
exposer le groupe réactif ayant le groupe protecteur à l'acide dans des conditions appropriées de sorte que le groupe protecteur est éliminé pour fournir un monomère ayant un groupement réactif exposé, dans lequel l'étape d'exposition ne se traduit pas par une dégradation substantielle des polymères de la matrice.

2. Procédé selon la revendication 1, dans lequel la matrice de polymères comprend une matrice d'acides nucléiques.

3. Procédé selon la revendication 2, dans lequel la matrice d'acides nucléiques comprend une matrice d'oligonucléotides.

4. Procédé selon la revendication 1, dans lequel le groupe réactif comprend une molécule choisie dans le groupe constitué par un agent de liaison, un monomère et un polymère.

5. Procédé selon la revendication 4, dans lequel la molécule est un monomère comprenant un nucléotide.

6. Procédé selon la revendication 5, dans lequel le nucléotide est protégé au niveau de son extrémité hydroxyle 5' par un groupe protecteur DMT.

7. Procédé selon la revendication 5, dans lequel le nucléotide est protégé au niveau de son groupe hydroxyle 3' par un groupe protecteur DMT.

8. Procédé selon la revendication 5, dans lequel le nucléotide est choisi dans le groupe constitué par G, A, T et C.

9. Procédé selon la revendication 8, dans lequel le nucléotide choisi dans le groupe constitué par G, A, T et C est protégé au niveau de son groupe hydroxyle 5' par un groupe protecteur DMT.

10. Procédé selon la revendication 4, dans lequel la molécule est un monomère comprenant un acide aminé.

11. Procédé selon la revendication 10, dans lequel l'acide aminé est un acide aminé naturel ou un acide aminé non naturel.

12. Procédé selon la revendication 10, dans lequel l'acide aminé est choisi dans le groupe constitué par l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la proline, la sérine, la thréonine, le tryptophane, la tyrosine et la valine.

13. Procédé selon la revendication 10, dans lequel l'acide aminé est protégé au niveau de sa fonctionnalité amino par un groupe protecteur tBoc.

14. Procédé selon l'une quelconque des revendications 1 à 13 dans lequel la lumière a une longueur d'onde comprise entre 330 et 365 nm.

15. Procédé selon la revendication 4, dans lequel le polymère est un acide nucléique et le monomère est un nucléotide et une détérioration substantielle est déterminée en déterminant le taux de faux négatifs engendrés par hybridation de la matrice avec un échantillon ayant des acides nucléiques complémentaires connus par rapport à la matrice.

16. Procédé selon la revendication 1, dans lequel le polymère est un peptide.

17. Procédé selon la revendication 1, comprenant en outre les étapes consistant à éliminer le film à partir du substrat avec un solvant approprié après l'élimination du groupe protecteur pour donner un substrat partiellement fini, comprenant un monomère ayant un groupe réactif exposé ;
faire réagir le monomère ayant un groupe réactif exposé avec un second monomère ayant un groupe réactif protégé par un groupe protecteur ; et
répéter les étapes de revêtement, d'activation d'exposition, d'élimination et de réaction pour fournir la matrice de polymère souhaitée.

18. Procédé selon la revendication 17 dans lequel le groupe réactif comprend un nucléotide ayant un groupe protecteur DMT.

19. Procédé selon la revendication 18, dans lequel le générateur de photo acide est choisi dans le groupe constitué par un générateur de photo acide ionique et un générateur non ionique.

20. Procédé selon la revendication 19, dans lequel le générateur de photo acide est un générateur de photo acide ionique.

21. Procédé selon la revendication 20 dans lequel le générateur de photo acide ionique est un sel d'onium.

22. Procédé selon la revendication 21 dans lequel le sel d'onium est le PF₆⁻ de Bis(4-t-butylphényl)iodonium.

23. Procédé selon la revendication 19 dans lequel le générateur de photo acide est un générateur de photo acide non ionique.

24. Procédé selon la revendication 23 dans lequel le générateur de photo acide non ionique est 2,6-dinitrobenzyle tosylate.

25. Procédé selon la revendication 1, dans lequel le film est constitué de poly(méthylméthacrylate).

26. Procédé selon la revendication 1, dans lequel le générateur de photo acide est un sel d'onium.

27. Procédé selon la revendication 26 dans lequel le sel d'onium est le PF₆⁻ de Bis(4-t-butylphényl)iodonium.

28. Procédé selon la revendication 27 dans lequel le sensibilisateur est le 2-isopropyle thioxanthone.

29. Procédé selon la revendication 1, dans lequel la matrice de polymères comprend un polymère ayant une longueur d'au moins 50 monomères.

30. Procédé selon la revendication 29, dans lequel la matrice de polymères comprend un polymère ayant une longueur d'au moins 60 monomères.

31. Procédé selon la revendication 30 dans lequel la matrice de polymères comprend un polymère ayant une longueur d'au moins 70 nucléotides.

32. Procédé selon la revendication 31, dans lequel le polymère est un oligonucléotide d'ADN.

33. Procédé selon la revendication 30 dans lequel le polymère est un oligonucléotide d'ADN.

34. Procédé selon la revendication 29 dans lequel le polymère est un oligonucléotide d'ADN.
